# FASCICULE DE BREVET EUROPEEN

(11) **EP 1 446 077 B1**
(45) Date de publication et mention de la délivrance du brevet: **19.12.2018**
(21) Numéro de dépôt: 02795347.0
(22) Date de dépôt: 25.10.2002
(51) Int. Cl.: A61F 5/56

(54) **ORTHESE INTRA-ORALE ANTI-RONFLEMENT**
INTRAORALE ORTHESE ZUM VERHINDERN DES SCHNARCHENS
INTRAORAL ORTHOSIS FOR PREVENTING SNORING

(30) Priorité: 26.10.2001 FR 0113918
(43) Date de publication de la demande: 18.08.2004
(60) Demande divisionnaire: 10195292.7
(73) Titulaire: ResMed SAS, 69791 Saint Priest Cedex (FR)
(72) Inventeur: Philipe Mousselon, 69210 Saint Germain sur l'Abresle (FR); Ludovic Baratier, 69004 Lyon (FR)
(74) Mandataire: Vossius & Partner Patentanwälte Rechtsanwälte mbB
(86) Numéro de dépôt international: PCT/FR2002/003684
(87) Numéro de publication internationale: WO 2003/034957

(56) Documents cités:
- DE-U- 20 102 432
- DE-U- 29 506 512
- US-A- 4 472 139
- US-A- 6 012 920

## Description

La présente invention concerne une orthèse intra-orale de prévention du ronflement et des apnées obstructives du sommeil.

Le ronflement chronique est une pathologie qui affecte une proportion considérable de la population estimée à 40% par certaines études.

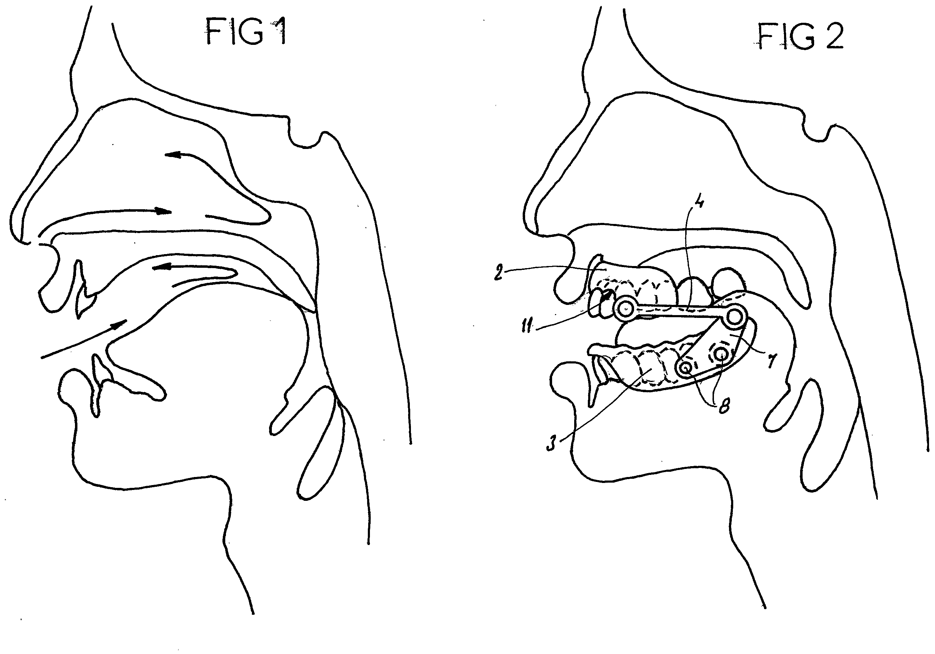

Pendant le sommeil, il se produit chez le patient un relâchement musculaire dans la zone de la gorge provoquant un rétrécissement du pharynx comme cela est représenté schématiquement sur la figure 1.

La conséquence de ce rétrécissement est une augmentation de la vitesse de l'air inspiré par un effet de type venturi. L'air excite la partie souple du voile du palais et la luette qui se mettent à vibrer bruyamment. Le bruit ainsi créé peut atteindre jusqu'à 90 décibels.

Dans certains cas, le rétrécissement est tel que la respiration peut être suspendue pendant une dizaine de secondes, voire plus pour certains patients.

Ce phénomène, appelé apnée du sommeil, conduit le patient à reprendre brutalement et bruyamment sa respiration.

Il existe, donc, de nombreux dispositifs permettant de prévenir le ronflement.

L'un des plus efficaces est une orthèse intra-orale qui réalise une avancée de la mâchoire inférieure. Cette avancée de la mâchoire inférieure permet un élargissement de la zone pharyngienne.

Du fait de la disparition du rétrécissement dû au relâchement musculaire, l'air est inspiré à une vitesse normale et les vibrations des parties souples du voile du palais et de la luette disparaissent.

Parmi ce type d'orthèse, il en existe qui comprennent deux gouttières thermoformées, dont l'une est destinée à chausser les dents de la mâchoire supérieure et l'autre à chausser les dents de la mâchoire inférieure.

Ces deux gouttières sont reliées latéralement par deux tirants.

Les tirants sont fixés symétriquement sur la gouttière supérieure au niveau des canines et sur la gouttière inférieure au niveau des premières molaires, la longueur des tirants étant telle que, lorsque l'orthèse est placée dans la bouche d'un patient sa mâchoire inférieure est maintenue en position avancée.

Les orthèses de ce type réduisent significativement les ronflements et sont bien tolérées par les patients. Toutefois, elles présentent certaines limitations.

On observe notamment de nombreux cas de décrochage des gouttières lorsque le patient ouvre la bouche inconsciemment dans son sommeil.

Lors de l'écartement des mâchoires l'une par rapport à l'autre, du fait de la liaison des gouttières entre elles par les tirants, les gouttières se décrochent des mâchoires et l'orthèse est expulsée de la bouche.

Le patient n'est alors plus traité et est donc de nouveau sujet aux ronflements.

Un autre inconvénient lié à ces orthèses tient dans le fait que le réglage de la longueur des tirants est fastidieux à réaliser puisqu'il faut démonter puis remonter complètement l'orthèse avec des tirants de longueur différente. Or la longueur des tirants est un paramètre critique de ce type d'orthèse puisque des tirants trop longs produisent une avancée insuffisante de la mâchoire inférieure tandis que des tirants de trop petite longueur ne sont pas supportés par le patient.

Le document US 6 012 920 montre un dispositif oral comportant deux gouttières sur lesquelles sont positionnés des boîtiers. Pour traiter d'apnée et de désordre du sommeil, les boîtiers supérieurs sont disposés de manière adjacente aux deuxièmes prémolaires tandis que les boîtiers inférieurs sont disposés de manière adjacente aux canines. Ce dispositif oral agit en poussant la mâchoire inférieure.

Un but de l'invention est donc de fournir une orthèse intra-orale de prévention du ronflement qui présente une meilleure tenue sur les mâchoires d'un patient.

Un autre but de l'invention est de fournir une orthèse permettant un réglage aisé de l'avancée de la mâchoire inférieure.

De manière connue en soi, l'orthèse comprend
- une gouttière supérieure et une gouttière inférieure destinées à revêtir respectivement une denture d'une mâchoire supérieure et une denture d'une mâchoire inférieure,
- deux tirants, reliant les gouttières, ces tirants étant d'une longueur telle que la mâchoire inférieure est maintenue dans une position avancée par rapport à la mâchoire supérieure,

Selon l'invention, les tirants présentent des points de fixation aux gouttières fixés,
- d'une part, à la gouttière supérieure au niveau des canines et,
- d'autre part à la gouttière inférieure au niveau de la deuxième molaire mandibulaire, la gouttière inférieure comprenant des moyens de fixation des tirants, permettant de déporter le point de fixation des tirants dans le plan occlusal de contact des dentures inférieure et supérieure.

Les tirants qui exercent une traction sur la gouttière inférieure, et donc sur la mâchoire inférieure, sont situés paralllement au plan auriculo-orbitaire dit plan de Francfort. Grâce à cette disposition, la traction des tirants se fait selon une composante quasiment parallèle au plan d'occlusion. L'orthèse est donc sensiblement moins sujette au décrochement.

Selon un mode de réalisation préféré, la gouttière inférieure est munie de pattes de fixation comprenant une partie fixée par deux rivets en regard des première et deuxième molaires et une partie s'étendant obliquement à environ 120° en direction de la face occlusale de la deuxième molaire.

Grâce à cette disposition des pattes, le point de fixation sur la gouttière inférieure de chacun des tirants est déporté dans une position postérieure, qui autorise l'utilisation de tirant de plus grande longueur. Ces tirants, dont la longueur est maximisée, confèrent à l'orthèse une très bonne tenue sur les mâchoires notamment en cas d'ouverture de la bouche.

De façon avantageuse, la gouttière supérieure s'étend sur un arc dont les extrémités s'arrêtent au niveau de la face distale des deuxièmes prémolaires maxillaires.

Grâce à cette disposition, la gouttière supérieure ne recouvrant pas les molaires est nettement moins envahissante que les gouttières connues. Cette découpe permet d'éviter un contact prématuré des gouttières maxillaire et mandibulaire au niveau des faces occlusales des molaires.

De plus, les gouttières présentent chacune dans leur paroi une découpe au niveau des incisives.

Dans une possibilité avantageuse, une liaison à rotule assure la liaison entre les tirants et les gouttières.

Selon une variante de réalisation, les tirants présentent des moyens permettant le réglage de leur longueur.

Pour un réglage aisé, les tirants présentent deux perçages filetés dans lesquels se visse une tige dont les extrémités présentent un pas inversé, un écrou étant placé au centre de la tige.

En agissant sur la tige, il est possible d'allonger ou de raccourcir les tirants sans devoir les démonter de l'orthèse.

Selon une forme de réalisation, les tirants comprennent une cavité cylindrique dans laquelle coulisse, sous l'action d'une pression hydraulique, une tige dont l'extrémité comprend un piston.

En outre, le piston délimite deux chambres reliées chacune par un conduit souple à deux vérins.

En agissant sur l'un ou l'autre des vérins, il est possible d'agir sur l'orthèse lorsque celle-ci est positionnée dans la bouche d'un patient afin de déterminer la longueur optimale des tirants.

Selon une autre possibilité, les tirants comprennent deux barrettes coulissant l'une dans l'autre, chacune étant pourvue de perçages.

Pour sa bonne compréhension, l'invention est décrite en référence au dessin représentant, à titre d'exemple non limitatif, une orthèse intra-orale selon celle-ci.
La figure 1 représente schématiquement une coupe sagittale de la cavité nasale, de la cavité buccale et de la région du pharynx d'un patient sujet au ronflement.
La figure 2 et 3 sont des vues de côté d'une orthèse positionnée sur des mâchoires inférieures et supérieures.
La figure 4 est une vue en perspective éclatée des moyens de fixation d'un tirant de cette orthèse.
Les figures 5 à 7 représentent des variantes de réalisation de ce tirant.
La figure 8 représente une forme de réalisation de tirant réglable.
Les figures 9 et 10 représentent deux formes de réalisation de liaison des tirants sur l'orthèse

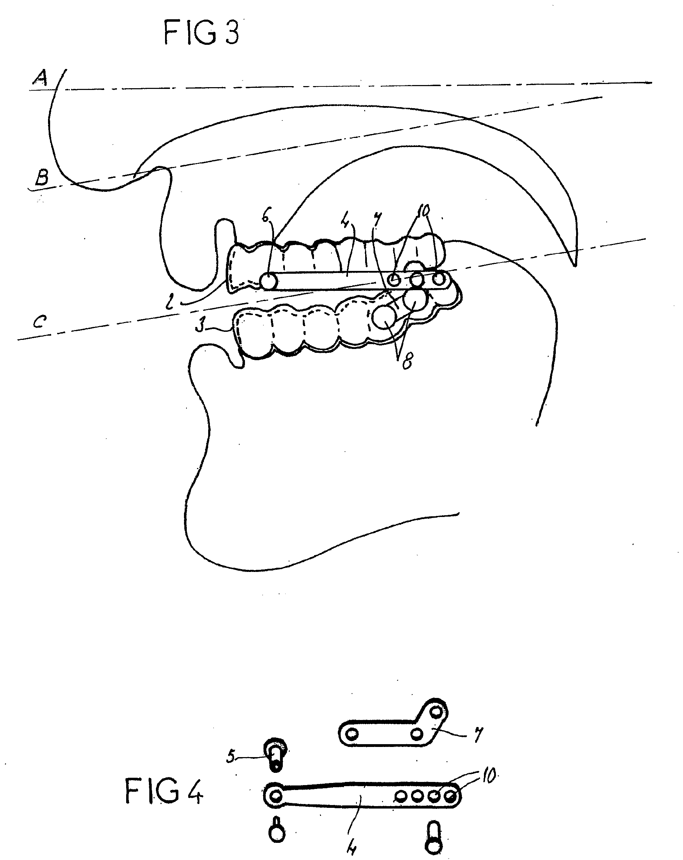

En se référant aux figures 2 et 3, on peut voir que l'orthèse destinée à prendre place dans la bouche d'un patient comprend une gouttière supérieure 2 et une gouttière inférieure 3 reliées par deux tirants 4.

Les plans référencés A, B, C représentent respectivement le plan de Francfort, le plan de Camper et le plan d'occlusion.

Les gouttières 2, 3 sont réalisées par thermoformage suivant les caractéristiques morphologiques des mâchoires du patient en vue de s'adapter parfaitement sur celles-ci.

Selon une possibilité, le matériau de thermoformage pourra être constitué de deux matières coextrudées une couche dure et une couche souple apportant un bon confort et présentant un effet ventouse qui contribue à la bonne tenue des gouttières.

La gouttière supérieure 2 présente au niveau de chacune des canines une liaison pivot réalisée par un rivet 5 avec l'extrémité supérieure de chacun des tirants 4.

La gouttière inférieure 3 est munie de deux pattes 7 présentant une extrémité libre sur laquelle s'articule l'extrémité inférieure de chacun des tirants 4.

Chacune des pattes 7 comprend une partie retenue sur la gouttière inférieure 3 par deux rivets 8, ces rivets 8 étant disposés en regard des première et deuxième molaires et une partie s'étendant obliquement à environ 120° en direction de la face occlusale de cette gouttière. Cet angle de 120° permet à l'extrémité de la patte 7 de se situer au niveau de la face occlusale de la deuxième molaire mandibulaire.

Les tirants 4 sont donc articulés par une liaison pivot sur la gouttière supérieure 2 et par une liaison pivot sur l'extrémité de la patte 7.

Par ailleurs, l'extrémité des tirants 4 situés du côté de la gouttière inférieure 3 est percée de quatre trous 10, permettant de régler la longueur de ces derniers.

Comme on peut le voir sur la figure 2, la gouttière supérieure 2 recouvre la mâchoire sur un arc s'arrêtant au niveau de la face distale des deuxièmes prémolaires.

Un patient sujet au ronflement est invité à chausser l'orthèse, les gouttières 2, 3 venant se superposer sur les mâchoires supérieure et inférieure.

Les tirants 4, qui exercent une traction sur la gouttière inférieure 3 et donc sur la mâchoire inférieure, sont situés parallèlement au plan de Francfort, et quasiment dans le plan de contact des dentures inférieure et supérieure.

Le positionnement des tirants 4 dans ce plan est rendu possible par le fait que l'extrémité inférieure des tirants 4 est fixée sur un point relevé au-dessus de la face occlusale des gouttières.

Grâce à cette disposition, la traction des tirants 4 se fait selon une composante parallèle au plan de Francfort.

La limitation de la composante de traction verticale apporte une très bonne tenue des gouttières 2,3 puisqu'une traction selon une composante verticale trop importante entraîne un risque important de décrochage des gouttières 2,3.

En outre, le déport du point d'articulation inférieure des tirants 4 au niveau de la deuxième molaire permet de mettre en oeuvre des tirants 4 présentant une longueur maximisée si bien que lorsque le patient ouvre la bouche durant son sommeil, les tirants 4 s'orientent selon une direction dont la composante horizontale reste très supérieure à la composante verticale.

Il ne se produit donc pas de décrochage des gouttières.

En outre, il faut noter que la gouttière supérieure 2 qui ne recouvre pas les molaires est nettement moins envahissante que les gouttières connues. Cette découpe permet d'éviter un contact prématuré des gouttières au niveau des faces occlusales des molaires maxillaire et mandibulaire.

On note également que les gouttières peuvent présenter des découpes 11 au niveau des incisives, dans le but de réaliser une orthèse la moins envahissante possible.

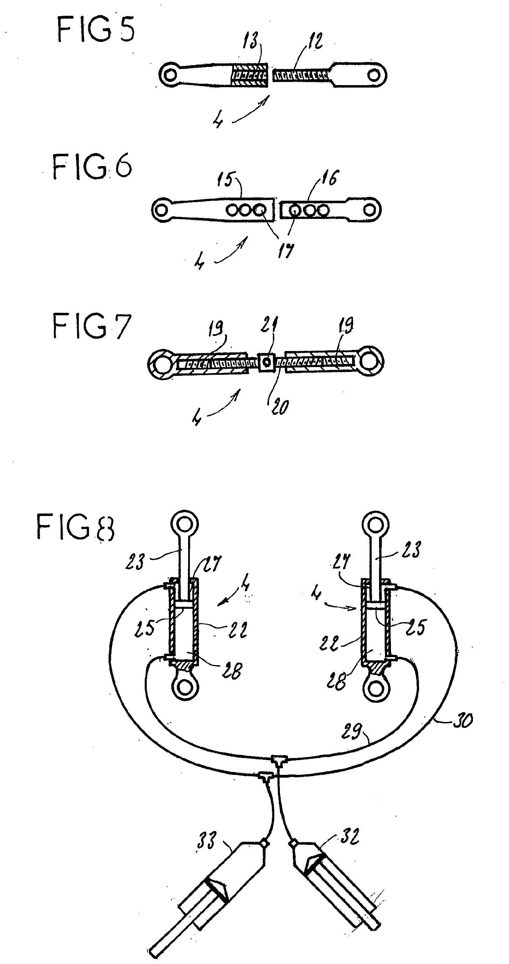

Les figures 5 à 7 représentent des formes de réalisation des tirants 4 permettant le réglage de leur longueur, de façon simple.

Le tirant représenté sur la figure 5 comprend deux éléments dont l'un présente une tige filetée 12 et l'autre un alésage fileté 13 destiné à recevoir la tige 12.

En vissant ou dévissant l'un des éléments par rapport à l'autre, le patient peut agir sur la longueur du tirant 4 et ajuster celui-ci en fonction de son ronflement ou de sa tolérance.

La figure 6 montre une autre possibilité de réalisation des tirants 4 dans laquelle les tirants 4 comprennent deux barrettes 15, 16 qui coulissent l'une dans l'autre et sont pourvues de trous 17 permettant de régler la longueur des tirants 4.

Sur la figure 7, on peut voir que les tirants 4 présentent deux perçages filetés 19 dans lesquelles se visse une tige 20 dont les extrémités présentent un pas inversé. Un écrou 21 placé au centre de la tige 20 permet de faire tourner celle-ci afin d'allonger ou de raccourcir les tirants 4 sans devoir démonter les tirants 4 de l'orthèse.

La figure 8 montre une variante de réalisation de l'orthèse adaptée à la réalisation d'examen clinique en vue de déterminer la longueur optimale des tirants 4.

Comme on peut le voir en coupe sur cette figure, les tirants 4 comprennent chacun un cylindre 22 dans lequel coulisse une tige 23.

L'extrémité libre de la tige 23 est équipée d'un piston 25 qui délimite deux chambres 27, 28 reliées chacune par des conduits 29, 30 souples à un dispositif permettant d'exercer une pression hydraulique sur le piston 25.

Ces dispositifs sont constitués, sur l'exemple représenté, par des vérins 32 permettant d'exercer une pression dans l'une ou l'autre des chambres 27, 28, et par voie de conséquence de déplacer la tige 23 de chacun des tirants 4, et donc d'allonger ou de raccourcir la longueur de ces derniers.

Dans certains cas, les patients atteints par un ronflement particulièrement aigu sont hospitalisés et les paramètres de leur sommeil sont analysés.

L'orthèse est donc placée dans la bouche du patient et les paramètres polysomnographiques du patient sont enregistrés.

En agissant sur l'une ou l'autre des vérins 32, il est possible d'agir sur l'orthèse in-situ et les enregistrements permettent de déterminer la longueur optimale des tirants 4 qui peuvent présenter des longueurs différentes en fonction de la morphologie du patient.

En effet, chacun des vérins est reliés par un raccord en Y ou T aux deux tirants si bien qu'il se produit un équilibrage des pressions régnant dans le tirant de droite et le tirant de gauche, cet équilibrage se faisant en fonction des forces particulières engendrées par le système neuro-musculaire du patient.

Il est ainsi possible de déterminer précisément la longueur optimale des tirants gauche et droit lors de l'enregistrement polysomnographique, ces tirants pouvant présenter des longueurs différentes puisque le corps humain n'est pas symétrique.

Il est également possible de prévoir un unique vérin 33 relié par un raccord en Y ou T à la chambre 27 de chaque tirant 4 de façon à agir sur la traction appliquée à la mandibule. Il est à noter que bien que ce ne soit pas représenté sur la figure 8, on peut prévoir en variante que des moyens mécaniques tels que des câbles réalisent le réglage de la longueur des tirants 4 afin d'éviter de placer dans la bouche d'un patient un appareillage hydraulique dans lequel un fluide sous haute pression circule, avec des risques de fuite dont les conséquences pourraient être très dommageables pour la cavité buccale.

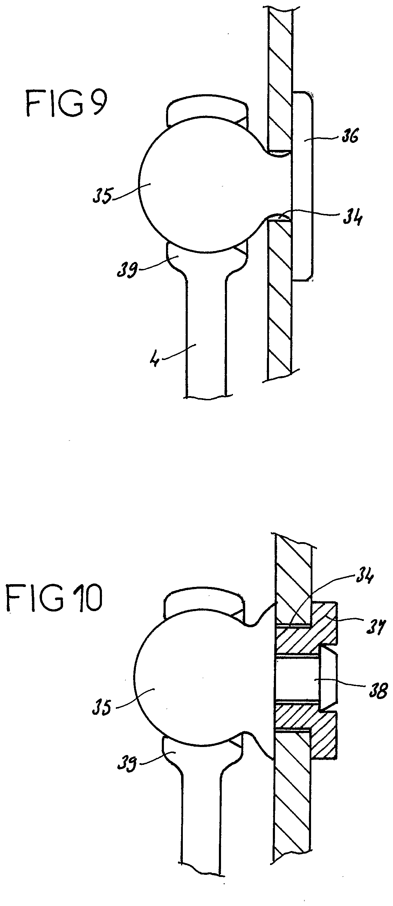

En se reportant aux figures 9 et 10, on observe que les tirants peuvent être articulés sur les gouttières par des rotules. A cet effet, comme cela apparaît à la figure 9, un perçage 34 est pratiqué dans la paroi de la gouttière à travers laquelle une rotule 35 est engagée, la rotule étant retenue par une pastille 36. La liaison représentée à la figure 10 met en oeuvre un insert 37 qui est engagé dans le perçage 34. La rotule 35 présente un pion 38 qui vient dans s'encliqueter dans l'insert 37. Dans ces deux variantes de réalisation, les tirants présentent un oeil 35 qui vient s'engager sur la rotule 35.

L'invention fournit ainsi une orthèse intra-orale ayant les nombreux avantages indiqués plus haut. Cette orthèse présente une excellente tenue sur les arcades dentaires grâce à la maximisation de la longueur des tirants. En outre, grâce à ses tirants réglables, elle peut être ajustée de manière optimale à la morphologie de chaque patient.

Il va de soi que l'invention n'est pas limitée à la forme de réalisation décrite ci-dessus à titre d'exemple mais qu'elle en embrasse, au contraire, toutes les variantes de réalisation. Ainsi, le réglage de la longueur des tirants pourrait se faire par une crémaillère dans laquelle une languette flexible s'encliquete. En outre, des moyens de blocage pourraient être prévus sur les tirants représentés sur la figure 7, permettant de fixer la longueur de ceux-ci dans leur position optimale déterminée de manière expérimentale. On pourrait également prévoir de solidariser les rivets 8 entre eux afin, d'une part de rigidifier la gouttière et, d'autre part d'obtenir une meilleure répartition des efforts de traction sur la gouttière supportant la patte.

## Revendications

1. Orthèse intra-orale comprenant
- une gouttière supérieure (2) et une gouttière inférieure (3) destinées à revêtir respectivement une denture d'une mâchoire supérieure et une denture d'une mâchoire inférieure,
- deux tirants (4), reliant les gouttières (2,3), ces tirants (4) étant d'une longueur telle que la mâchoire inférieure est maintenue dans une position avancée par rapport à la mâchoire supérieure,
**caractérisée en ce que** les tirants (4) présentent des points de fixation aux gouttières (2,3) fixés,
- d'une part, à la gouttière supérieure (2) au niveau des canines et,
- d'autre part à la gouttière inférieure (3) au niveau de la deuxième molaire mandibulaire, la gouttière inférieure (3) comprenant des moyens de fixation des tirants (4), permettant de déporter le point de fixation des tirants (4) dans le plan occlusal de contact des dentures inférieure et supérieure.

2. Orthèse selon la revendication 1, **caractérisée en ce que** les tirants (4) sont fixés à la gouttière inférieure sur un point de fixation relevé au-dessus de la face occlusale des gouttières.

3. Orthèse selon l'une des revendications 1 à 2, **caractérisée en ce que** la gouttière inférieure (3) est munie de pattes (7) de fixation comprenant une partie fixée par deux rivets en regard des première et deuxième molaires et une partie s'étendant obliquement à environ 120° en direction de la face occlusale de la deuxième molaire.

4. Orthèse selon l'une des revendications 1 à 3, **caractérisée en ce que** la gouttière supérieure (2) s'étend sur un arc dont les extrémités s'arrêtent en arrière des deuxièmes prémolaires maxillaires.

5. Orthèse selon l'une des revendications 1 à 4, **caractérisée en ce que** les gouttières (2,3) présentent chacune dans leur paroi une découpe (11) dégageant les incisives centrales et latérales.

6. Orthèse selon l'une des revendications 1 à 5, **caractérisée en ce que** une liaison à rotule assure la liaison entre les tirants (4) et les gouttières.

7. Orthèse selon l'une des revendications 1 à 4, **caractérisée en ce que** les tirants (4) présentent des moyens permettant le réglage de leur longueur.

8. Orthèse selon la revendication 6, **caractérisée en ce que** les tirants présentent deux perçages filetés (19) dans lesquels se visse une tige (20) dont les extrémités présentent un pas inversé, un écrou (21) étant placé au centre de la tige (20).

9. Orthèse selon la revendication 6, **caractérisée en ce que** les tirants (4) comprennent un cylindre (22) dans laquelle coulisse sous l'action d'une pression hydraulique, une tige (23) dont l'extrémité comprend un piston (25).

10. Orthèse selon la revendication 6, **caractérisée en ce que** le piston (25) délimite deux chambres (27,28) reliées chacune par un conduit (29,30) souple à deux vérins (32,33).

11. Orthèse selon la revendication 6, **caractérisée en ce que** les tirants comprennent deux barrettes (15,16) coulissant l'une dans l'autre, chacune étant pourvue de perçages.

12. Orthèse selon la revendication 6, **caractérisée en ce que** un vérin unique est relié, par deux conduits, à deux tirants (4) dont la longueur est réglable en fonction de la charge appliquée par le vérin.

13. Orthèse selon l'une des revendications 1 à 12, **caractérisée en ce que** les gouttières sont réalisées en un matériau bi-composants comprenant un matériau dur et un matériau souple.

## Patentansprüche

1. Intraorale Orthese, aufweisend:
- eine an Zähne eines Oberkiefers anzulegende obere Schiene (2) und eine an Zähne eines Unterkiefers anzulegende untere Schiene (3) und
- zwei Zugglieder (4), die die Schienen (2,3) miteinander verbinden, wobei diese Zugglieder (4) von einer solchen Länge sind, dass der Unterkiefer in einer bezüglich des Oberkiefers vorgeschobenen Position gehalten wird,
**dadurch gekennzeichnet, dass** die Zugglieder (4) Befestigungspunkte aufweisen, um sie mit den Schienen (2,3) zu verbinden, nämlich
- einerseits mit der oberen Schiene (2) im Bereich der Eckzähne und
- andererseits mit der unteren Schiene (3) im Bereich der zweiten Molaren des Unterkiefers,
wobei die untere Schiene (3) Befestigungsmittel zum Befestigen der Zugglieder (4) aufweist, die erlauben, den Befestigungspunkt der Zugglieder (4) in die Ebene des Okklusionskontakts der unteren und oberen Zahnreihe zu verlegen.

2. Orthese nach Anspruch 1, **dadurch gekennzeichnet, dass** die Zugglieder (4) an der unteren Schiene an einem Befestigungspunkt befestigt sind, der oberhalb der Schließfläche der Schienen angeordnet ist.

3. Orthese nach einem der Ansprüche 1 bis 2, **dadurch gekennzeichnet, dass** die untere Schiene (3) mit Halteklammern (7) versehen ist, die einen dem ersten und zweiten Backenzahn gegenüberliegenden, mit zwei Nieten befestigten Abschnitt und einen sich etwa 120° schräg in Richtung der Okklusionsfläche des zweiten Molars erstreckenden Abschnitt aufweisen.

4. Orthese nach einem der Ansprüche 1 bis 3, **dadurch gekennzeichnet, dass** die obere Schiene (2) sich in einem Bogen erstreckt und mit ihren Enden bis an die Rückseite der zweiten Prämolaren reicht.

5. Orthese nach einem der Ansprüche 1 bis 4, **dadurch gekennzeichnet, dass** die Schienen (2,3) jeweils in ihrer Wand eine Aussparung (11) aufweisen, so dass die zentralen und lateralen Schneidezähne freiliegen.

6. Orthese nach einem der Ansprüche 1 bis 5, **dadurch gekennzeichnet, dass** eine Kugelgelenkverbindung die Verbindung zwischen den Zuggliedern (4) und den Schienen bereitstellt.

7. Orthese nach einem der Ansprüche 1 bis 4, **dadurch gekennzeichnet, dass** die Zugglieder (4) Mittel aufweisen, die die Anpassung ihrer Länge erlauben.

8. Orthese nach Anspruch 6, **dadurch gekennzeichnet, dass** die Zugglieder zwei Gewindebohrungen (19) aufweisen, in die eine Stange (20) geschraubt ist, deren Enden ein Gegengewinde aufweisen, wobei eine Schraubenmutter (21) in der Mitte der Stange (20) angeordnet ist.

9. Orthese nach Anspruch 6, **dadurch gekennzeichnet, dass** die Zugglieder (4) einen Zylinder (22) aufweisen, in welchem unter der Wirkung eines hydraulischen Drucks eine Stange (23) gleitet, deren Ende einen Kolben (25) aufweist.

10. Orthese nach Anspruch 6, **dadurch gekennzeichnet, dass** der Kolben (25) zwei Kammern (27, 28) begrenzt, die jeweils über eine flexible Leitung (29, 30) mit zwei Betätigungseinrichtungen (32, 33) verbunden sind.

11. Orthese nach Anspruch 6, **dadurch gekennzeichnet, dass** die Zugglieder zwei ineinander verschiebbare Stäbe (15, 16) aufweisen, die jeweils mit Bohrungen versehen sind.

12. Orthese nach Anspruch 6, **dadurch gekennzeichnet, dass** eine einzige Betätigungseinrichtung über zwei Leitungen mit zwei Zuggliedern (4) verbunden ist, deren Länge abhängig von der Last, die von der Betätigungseinrichtung ausgeübt wird, anpassbar ist.

13. Orthese nach einem der Ansprüche 1 bis 12, **dadurch gekennzeichnet, dass** die Schienen aus einem zweikomponentigen Material hergestellt sind, das ein hartes Material und ein weiches Material aufweist.

## Claims

1. An intraoral orthosis comprising
- an upper splint (2) and a lower splint (3) designed to line teeth of an upper jaw and teeth of a lower jaw, respectively,
- two tie rods (4) connecting the splints (2, 3), these tie rods (4) having such a length that the lower jaw is maintained in an advanced position relative to the upper jaw, **characterized in that** the tie rods (4) have fixed points of attachment to the splints (2, 3)
- on the one hand, to the upper splint (2) at the canines, and,
- on the other hand, to the lower splint (3) at the second mandibular molar, wherein the lower splint (3) comprises means for fixation of the tie rods (4) permitting to shift the point of attachment of the tie rods (4) in the occlusal plane of contact of the lower and upper teeth.

2. The orthosis according to claim 1, **characterized in that** the tie rods (4) are fixed to the lower splint on a point of attachment raised above the occlusal face of the splints.

3. The orthosis according to any one of claims 1 to 2, **characterized in that** the lower splint (3) is equipped with attachment brackets (7) comprising a part fixed by two rivets in line with the first and second molars, and a part extending obliquely at about 120° in the direction of the occlusal face of the second molar.

4. The orthosis according to any one of claims 1 to 3, **characterized in that** the upper splint (2) extends on an arc whose ends stop behind the second maxillary premolars.

5. The orthosis according to any one of claims 1 to 4, **characterized in that** the splints (2, 3) each have, in their wall, a cut-out (11) exposing the central and lateral incisors.

6. The orthosis according to any one of claims 1 to 5, **characterized in that** a ball-and-socket joint provides the connection between the tie rods (4) and the splints.

7. The orthosis according to any one of claims 1 to 4, **characterized in that** the tie rods (4) have means permitting the adjustment of their length.

8. The orthosis according to claim 6, **characterized in that** the tie rods have two threaded bores (19) into which a rod (20) whose ends have an inverse pitch is screwed, wherein a nut (21) is placed at the middle of the rod (20).

9. The orthosis according to claim 6, **characterized in that** the tie rods (4) comprise a cylinder (22) in which a rod (23) whose end comprises a piston (25) slides under the action of hydraulic pressure.

10. The orthosis according to claim 6, **characterized in that** the piston (25) delimits two chambers (27, 28) which are each connected via a flexible conduit (29, 30) to two actuators (32, 33).

11. The orthosis according to claim 6, **characterized in that** the tie rods comprise two small bars (15, 16) sliding one inside the other, wherein each is provided with holes.

12. The orthosis according to claim 6, **characterized in that** a single actuator is connected via two conduits to two tie rods (4) whose length is adjustable depending on the load applied by the actuator.

13. The orthosis according to any one of claims 1 to 12, **characterized in that** the splints are made of a two-component material comprising a hard material and a flexible material.
